# EUROPEAN PATENT APPLICATION

(11) **EP 2 700 402 A2**
(43) Date of publication of application: **26.02.2014**
(21) Application number: 12774686.5
(22) Date of filing: 20.04.2012
(51) Int. Cl.: A61K 31/12, A61K 31/05, A61P 25/28, A61P 3/06

(54) **NOVEL USES OF LICOCHALCONE A**

(30) Priority: 21.04.2011 KR 20110037180
(71) Applicant: University-Industry Cooperation Group of Kyung Hee University, Gyeonggi-do 446-701 (KR)
(72) Inventor: CHUNG, Sung Hyun, Seoul 137-040 (KR); QUAN, Hai Yan, Seoul 130-080 (KR)
(74) Representative: McConchie, Connor Fraser
(86) International application number: PCT/KR2012/003066
(87) International publication number: WO 2012/144854

(57) **Abstract**

The present invention relates to a pharmaceutical or food composition containing licochalcone A as an active ingredient for preventing, improving, or treating AMPK-related diseases, specifically, disorders involving lipid metabolism, hyperlipidemia, metabolic syndrome, obesity, fatty liver, or degenerative diseases (in particular, dementia). The pharmaceutical or food composition according to the present invention significantly reduces adipocyte differentiation, fat accumulation in adipocytes, and lipogenesis in hepatocytes, and exhibits the effects of activating AMPK in hepatocytes. Accordingly, the pharmaceutical or food composition of the present invention can be effectively used for AMPK-related diseases, specifically, disorders involving lipid metabolism, hyperlipidemia, metabolic syndrome, obesity, fatty liver, or degenerative diseases (in particular, dementia).

<110>	University-Industry Cooperation Group of Kyung Hee University

<120>	NOVEL USES OF LICOCHALCONE A

<160>	22

<170>	KopatentIn 1.71

<210>	1
<211>	20
<212>	DNA
<213>	Artificial Sequence

<220>
<223>	Primer for PPAR-gamma (Forward)


<400>	1
gcgctaccgg tcttctatca                                                    20


<210>	2
<211>	19
<212>	DNA
<213>	Artificial Sequence

<220>
<223>	Primer for PPAR-gamma (Reverse)


<400>	2
tgctgccaaa agacaagcg                                                     19


<210>	3
<211>	19
<212>	DNA
<213>	Artificial Sequence

<220>
<223>	Pirmer for C/EBP-alpha (Forward)


<400>	3
gatcctggaa cgagaacac                                                     19


<210>	4
<211>	21
<212>	DNA
<213>	Artificial Sequence

<220>
<223>	Primer for C/EBP-alpha (Reverse)


<400>	4
agactcgtgg aacacggtgg t                                                  21


<210>	5
<211>	22
<212>	DNA

<213>    Artificial Sequence

<220>
<223>    Primer for aP2 (Forward)


<400>    5
cgagggttgg ttgttgatct gt                                                22


<210>    6
<211>    20
<212>    DNA
<213>    Artificial Sequence

<220>
<223>    Primer for aP2 (Reverse)


<400>    6
atagcactgt tggccctgga                                                   20


<210>    7
<211>    20
<212>    DNA
<213>    Artificial Sequence

<220>
<223>    Primer for SREBP1 (Forward)


<400>    7
gcgctaccgg tcttctatca                                                   20


<210>    8
<211>    19
<212>    DNA
<213>    Artificial Sequence

<220>
<223>    Primer for SREBP1 (Reverse)


<400>    8
tgctgccaaa agacaaggg                                                    19


<210>    9
<211>    20
<212>    DNA
<213>    Artificial Sequence

<220>
<223>    Primer for SCD-1 (Forward)


<400>    9
ccctgaacat cgagtgtcga                                                   20


<210>    10

<211>    23
<212>    DNA
<213>    Artificial Sequence

<220>
<223>    Primer for SCD-1 (Reverse)


<400>    10
cttgcccaga gatttgaggt cct                                              23


<210>    11
<211>    23
<212>    DNA
<213>    Artificial Sequence

<220>
<223>    Primer for FAS (Forward)


<400>    11
ggtagtggat actctgtcgt cca                                              23


<210>    12
<211>    20
<212>    DNA
<213>    Artificial Sequence

<220>
<223>    Primer for FAS (Reverse)


<400>    12
catcagcaac atcattcggt                                                  20


<210>    13
<211>    23
<212>    DNA
<213>    Artificial Sequence

<220>
<223>    Primer for GPAT (Forward)


<400>    13
ggtagtggat actctgtcgt cca                                              23


<210>    14
<211>    17
<212>    DNA
<213>    Artificial Sequence

<220>
<223>    Primer for GPAT (Reverse)


<400>    14
cagcaacatc attcggt                                                     17

<210>    15
<211>    18
<212>    DNA
<213>    Artificial Sequence

<220>
<223>    Primer for CPT1 (Forward)


<400>    15
cctgggcatg attgcaag                                                    18


<210>    16
<211>    23
<212>    DNA
<213>    Artificial Sequence

<220>
<223>    Primer for CPT1 (Reverse)


<400>    16
acagactcca ggtacctgct cac                                              23


<210>    17
<211>    20
<212>    DNA
<213>    Artificial Sequence

<220>
<223>    Primer for beta-actin (Forward)


<400>    17
gtcgtaccac tggcattgtg                                                  20


<210>    18
<211>    20
<212>    DNA
<213>    Artificial Sequence

<220>
<223>    Primer for beta-actin (Reverse)


<400>    18
gccatctcct gctcaaagtc                                                  20


<210>    19
<211>    20
<212>    DNA
<213>    Artificial Sequence

<220>
<223>    Primer for PPAR-alpha


<400>    19

tccgactccg tcttcttgat                                                    20

<210>    20
<211>    21
<212>    DNA
<213>    Artificial Sequence

<220>
<223>    Primer for PPAR-alpha

<400>    20
gcctaaggaa accgttctgt g                                                  21

<210>    21
<211>    21
<212>    DNA
<213>    Artificial Sequence

<220>
<223>    Primer for CD36 (Forward)

<400>    21
gggctatagg gatccatttt t                                                  21

<210>    22
<211>    23
<212>    DNA
<213>    Artificial Sequence

<220>
<223>    Primer for CD36 (Reverse)

<400>    22
cctttcagat taacgtcgga ttc                                                23

## Description

### [Technical Field]

The present invention relates to novel uses of licochalcone A.

### [Background Art]

So-called "lifestyle-related diseases" such as obesity, hyperlipidemia, diabetes, hypertension, etc. are rapidly increasing due to changes in lifestyle, especially, the increase in fat intake, lack of exercise, etc. Here, obesity is a complex syndrome involving a variety of factors such as genetic, nutritional, environmental, and social factors. Hyperlipidemia, an increase in the amount of cholesterol and triglycerides in blood, occurs due to obesity, resulting in the development of hypertension, cardiovascular disease, stroke, etc. Moreover, excessive fat intake may cause an increase in hepatic lipogenesis, a decrease in release and breakdown of triglycerides, etc. due to high blood cholesterol and accumulation of fat in body, resulting in fatty liver, an accumulation of fat in the liver, as well as cardiovascular diseases such as arteriosclerosis, etc.

Most fat accumulated in a fatty liver is triglycerides, and fatty liver can be generally divided into alcoholic fatty liver disease due to excessive drinking and non-alcoholic fatty liver disease due to obesity, diabetes, hyperlipidemia, drugs, etc. Non-alcoholic fatty liver disease mainly occurs in people suffering from obesity, hyperinsulinemia, diabetes, etc. and is caused by an increase in the level of free fatty acid in blood due to insulin resistance or excessive lipolysis.

At present, there are almost no drugs available to pharmacologically treat fatty liver disease, and exercise and diet are only recommended. Thus, the development of a therapeutic agent for fatty liver disease is required.

Lipid metabolism disorders are caused by insulin resistance as well as excessive intake of fat or cholesterol. Both an increase in the level of blood LDL cholesterol or triglycerides in the blood and a decrease in the level of HDL cholesterol are the causes of atherosclerosis. Under these circumstances, the development of medicines and foods effective in improving lipid metabolism, suppressing fat accumulation, etc. is urgently required.

AMP-activated protein kinase (AMPK) is a protein that is activated by changes in the intracellular AMP:ATP ratio and is a representative protein that plays a key role in promoting energy metabolism by inhibiting ATP-consuming pathways and activating ATP-producing pathways. It is known that the activation of AMPK can increase fatty acid oxidation, suppress triglyceride accumulation in liver, and prevent or treat type 2 diabetes, metabolic syndrome, obesity, cancer, degenerative diseases (in particular, dementia), etc. [AMPK and the biochemistry of exercise: Implications for human health and disease. Biochem J 418(2), 261-275 (2009), Mechanisms linking obesity, chronic kidney disease, and fatty liver disease: the roles of fetuin-A, adiponectin, and AMPK. J Am Soc Nephrol 2010 Mar.; (21) 3: 406-12].

Sterol regulatory element-binding protein (SREBP) is synthesized as an inactive precursor bound to the endoplasmic reticulum membrane, activated when the N terminus is cleaved off, translocates to the nucleus, and binds to sterol regulatory element (SRE). Among them, it is known that SREBP1 mainly regulates the synthesis of triglycerides. Genes regulating lipogenesis includes fatty acid synthase (FAS), stearoyl-CoA desaturase 1 (SCD1), glycerol-3-phosphate acyltransferase (GPAT), etc. as well as SREBP1. It is known that peroxisome proliferator-activated receptor-γ (PPARγ) is an important transcription factor for adipocyte differentiation. Moreover, it is known that the interaction between PPARγ and C/EBP (CCAAT/enhancer-binding protein) is important for mature adipocytes and regulates the expression of adipocyte-specific proteins, such as adipocyte protein 2 (aP2), and lipid metabolic enzymes.

### [Disclosure]

### [Technical Problem]

The present invention provides a pharmaceutical or food composition for preventing or treating lipid metabolism disorder, hyperlipidemia, metabolic syndrome, obesity, or fatty liver disease.

### [Technical Solution]

Surprisingly, the present inventors have found that licochalcone A activates AMP-activated protein kinase (AMPK) in hepatocytes, stimulates lipolysis, inhibits lipogenesis, suppresses differentiation of preadipocytes into adipocytes, and inhibits lipogenesis in adipocytes and completed the present invention.

In an embodiment of the present invention, the present invention provides a pharmaceutical composition containing licochalcone A as an active ingredient for preventing or treating AMPK-related diseases.

In another embodiment of the present invention, the present invention provides a food composition containing licochalcone A as an active ingredient for preventing or treating AMPK-related diseases.

In this specification, the AMPK-related diseases refer to one or more diseases selected from the group consisting of lipid metabolism disorder, hyperlipidemia, metabolic syndrome, obesity, fatty liver disease, and degenerative diseases (in particular, dementia).

Licochalcone A {(2E)-3-[5-(1,1-dimethyl-2-propenyl)-4-hydroxy-2-methoxyphenyl]-1-(4-hdyroxyphenyl)-2-propen-1-one} as an active ingredient of the pharmaceutical or food composition of the present invention is one of the major flavonoids in licorice (Glycyrrhizae radix), and its chemical structure is as follows:

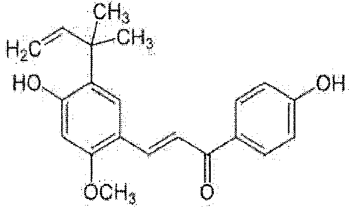

While physiological functions of licochalcone A have been unknown, the licochalcone A has been used as a potential antimalarial agent for a long time (Chen et al., Antimicrob Agents Chemother. 38(7) pp.1470-1475, 1994). Moreover, the anticancer effect (E.J. Park et al., Planta Med. 64, pp.464, 1998), the anti-inflammatory effect (Korean Patent Publication No. 10-2010-0077553), the prevention and treatment effect on diabetic nephropathy (Korean Patent Publication No. 10-2010-0135424), etc. have been known, but the AMPK activation effect and the treatment effect on lipid metabolism disorder, hyperlipidemia, metabolic syndrome, obesity, or fatty liver disease have not been reported.

In the present invention, the licochalcone A can be used in the form of a pharmaceutically acceptable salt thereof and can also be used in the form of a solvate or prodrug.

In the present invention, the licochalcone A may be isolated from natural materials such as licorice, turnips, etc. by a method well known in the art and may be artificially synthesized by a method commonly used by those skilled in the art.

The content of licochalcone A in the pharmaceutical composition of the present invention may be appropriately adjusted depending on the condition and severity of disease, the state of a patient, etc. and may be 1 to 50 wt%, preferably 1 to 5 wt%, with respect to the total weight of the composition, but not limited thereto.

The licochalcone A contained as an active ingredient in the pharmaceutical composition of the present invention may be formulated with pharmaceutically acceptable carriers, excipients, or diluents. For further information about the formulation, please refer to literatures such as Remington's Pharmaceutical Science (the latest version), Mack Publishing Company, Easton PA, etc.

In an embodiment of the present invention, the pharmaceutical composition containing licochalcone A according to the present invention may be formulated into oral dosage forms such as powders, granules, tablets, capsules, suspensions, emulsions, syrups, aerosols, etc., medicines for external use, suppositories, sterile injection solutions, etc.

Examples of the carriers, excipients, and diluents that can be contained in the pharmaceutical composition may include lactose, dextrose, sucrose, sorbitol, mannitol, xylitol, erythritol, maltitol, starch, acacia gum, alginate, gelatin, calcium phosphate, calcium silicate, cellulose, methyl cellulose, microcrystalline cellulose, polyvinylpyrrolidone, water, methyl hydroxybenzoate, propyl hydroxybenzoate, talc, magnesium stearate, mineral oil, etc., but not limited thereto.

Moreover, the pharmaceutical composition of the present invention may comprise generally-used diluents, excipients, or other pharmaceutically acceptable additives such as fillers, extenders, binders, wetting agents, disintegrating agents, surfactants.

Solid preparations for oral administration may include tablets, pills, powders, granules, capsules, etc. and may be prepared by mixing with one or more excipients such as starch, calcium carbonate, sucrose or lactose, gelatin, etc., in addition to the licochalcone A. Moreover, lubricants such as magnesium stearate, talc, etc. can be used in addition to simple excipients.

Liquid preparations for oral administration may include suspensions, solutions, emulsions, syrups, etc. and may further comprise various excipients such as wetting agents, sweetening agents, flavoring agents, preservatives, etc. in addition to commonly-used simple diluents such as water, liquid paraffin, etc.

Preparations for parenteral administration may include sterilized aqueous solutions, non-aqueous solvents, suspensions, emulsions, freeze drying agents, and suppositories. Examples of the non-aqueous solvents and suspensions may include propylene glycol, polyethylene glycol, vegetable oil such as olive oil, injectable ester such as ethylolate, etc., but not limited thereto. Basic materials of suppositories may include witepsol, macrogol, Tween 61, cacao butter, laurin butter, glycerinated gelatin, etc., but not limited thereto.

The preferred dosage of the pharmaceutical composition of the present invention depends on the condition and weight of a patient, the severity of disease, the type of drug, the route and duration of administration, etc., but may be a therapeutically effective amount appropriately selected by those skilled in the art. For the desired effect, the dosage of the composition of the present invention may be 10 mg/kg to 30 mg/kg per day for an adult male, preferably 5 mg/kg to 10 mg/kg, based on the content of licochalcone A as an active ingredient.

The daily dose may be administered once or several times a day. However, the scope of the present invention is not limited to the dosage and frequency of administration.

The pharmaceutical composition of the present invention may be administered to mammals such as rats, mice, cattle, and human via various routes such as oral administration or intraperitoneal, intrarectal, intravenous, intramuscular, subcutaneous, intrauterine, or intracerebroventricular injection.

The pharmaceutical composition of the present invention can be used for the prevention or treatment of AMPK-related diseases. That is, the pharmaceutical composition of the present invention can be used for the prevention or treatment of one or more diseases selected from the group consisting of lipid metabolism disorder, hyperlipidemia, metabolic syndrome, obesity, fatty liver disease, and degenerative diseases (in particular, dementia). Here, hyperlipidemia includes conditions associated with hyperlipidemia, and the conditions associated with hyperlipidemia include atherosclerosis, angina pectoris, carotid artery disease, cerebral arteriosclerosis, xanthoma, CHD, ischemic stroke, restenosis after angioplasty, peripheral vascular disease, intermittent claudication, myocardial infarction (e.g. reduction in necrosis), dyslipidemia, post-prandial lipemia, etc. The degenerative diseases may be dementia, preferably, Alzheimer-type dementia.

The present invention further provides uses of licochalcone A for preventing or treating the above-mentioned diseases.

The present invention further provides uses of licochalcone A for the preparation of medicines for preventing or treating the above-mentioned diseases.

The present invention further provides a method for preventing or treating the above-mentioned AMPK-related diseases, the method comprising administering a therapeutically effective amount of licochalcone A to a subject, e.g., a mammal including human, in need of preventing or treating the above-mentioned AMPK-related diseases.

The present invention further provides a food composition containing licochalcone A as an active ingredient for preventing or improving AMPK-related diseases. The food composition of the present invention can be used as a health functional food. As used herein, the term "health functional food" means foods manufactured and processed by using raw materials and ingredients that are beneficial for the human body in compliance with the Health Functional Food Act No. 6727. The term "functional" means that the intake of food is directed to controlling nutriments on the structure and function of a human body or achieving useful effects on health such as physiological effects.

The food composition of the present invention may contain any food additives. Unless otherwise specified, the suitability of food additives is determined by the specification and standard of the concerned item in compliance with General provisions and General Test Methods of the Korean Food Additives Codex approved by the Korean Food and Drug Administration.

Examples of the items mentioned in the "Korean Food Additives Codex" may include chemical compounds such as ketones, glycine, sodium citrate, nicotinic acid, cinnamic acid, etc., natural additives such as persimmon color, licorice extract, crystalline cellulose, kaoliang color, guar gum, etc., and mixed formulations such as L-sodium glutamate, alkali additives for noodles, preservatives, tar color formulation, etc.

The health functional food composition of the present invention can be variously used in foods and beverages for preventing or treating AMPK-related diseases, specifically, lipid metabolism disorder, hyperlipidemia, metabolic syndrome, obesity, fatty liver disease, or degenerative diseases (in particular, dementia). Moreover, the health functional food composition of the present invention can be used in various foods, beverages, gums, teas, vitamin complexes, health functional foods, food additives, etc. and can be used in the form of powders, granules, tablets, capsules, or beverages, or used in any other forms.

The food composition of the present invention may contain licochalcone A in an amount of 1 to 50 wt%, preferably 1 to 5 wt%, with respect to the total weight of the composition, for the purpose of preventing or improving AMPK-related diseases, specifically, lipid metabolism disorder, hyperlipidemia, metabolic syndrome, obesity, fatty liver disease, or degenerative diseases (in particular, dementia).

Moreover, the food composition of the present invention may be prepared and processed in the form of tablets, capsules, powders, liquids, pills, etc, for the purpose of preventing or improving AMPK-related diseases, specifically, lipid metabolism disorder, hyperlipidemia, metabolic syndrome, obesity, fatty liver disease, or degenerative diseases (in particular, dementia).

For example, the health functional food in the form of tablets may be prepared by granulating a mixture of licochalcone A, excipient, binder, disintegrating agent, and other additives by a general method, adding a glidant to the granules, and compression-molding the resulting granules, or the mixture may be directly compression-molded. Moreover, the health functional food in the form of tablets may contain a flavor enhancer, if necessary, and may be coated with an appropriate coating agent, if necessary.

The definitions of the terms such as excipient, binder, glidant, disintegrating agent, flavor enhancer, flavoring agent, etc. are described in references known in the art and include those having the same or similar functions (Handbook of the Korean Pharmacopoeia, MoonSung Co., Korean Association of Pharmacy Education, 5th edition, p33-48, 1989).

### [Advantageous Effects]

The licochalcone A of the present invention has the effects of activating AMPK in hepatocytes, stimulating lipolysis, inhibiting lipogenesis, suppressing differentiation of preadipocytes into adipocytes, and inhibiting lipogenesis in adipocytes. Thus, the pharmaceutical or food composition containing licochalcone A has the effects of preventing, improving, or treating AMPK-related diseases, specifically, lipid metabolism disorder, hyperlipidemia, metabolic syndrome, obesity, fatty liver disease, or degenerative diseases (in particular, dementia).

### [Description of Drawings]

FIG. 1 is a diagram showing the morphology of 3T3-L1 adipocytes after differentiation in non-treated group (con), pioglitazone-treated group (Pio), and licochalcone A-treated group.
FIG. 2 is a graph showing triglyceride levels at 540 nm after destaining.
FIG. 3 is a diagram showing the results of Western blotting to determine the effects of licochalcone A on the expression of transcription factors for adipocyte differentiation (PPARγ and C/EBPα) in 3T3-L1 cells.
FIG. 4 is a diagram showing the results of RT-PCR to determine the effects of licochalcone A on the expression of transcription factors for adipocyte differentiation (PPARγ and C/EBPα) in 3T3-L1 cells (FIG. 4A) and the expression of lipogenic genes (SREBP1, FAS, SCD1, and GPAT) and a fatty acid oxidation-related gene (CPT-1) (FIG. 4B).
FIG. 5 is a diagram showing the effects of licochalcone A on AMPK and ACC phosphorylation.
FIG. 6 showing the results of RT-PCR to determine the effects of licochalcone A on suppressing the expression of transcription factors for lipogenesis (SREBP1, SCD1, and FAS) and stimulating the expression of lipolytic genes (PPARα and CD36).

### [Mode for Invention]

Hereinafter, the present invention will be described in detail with reference to Examples and Experimental Examples. However, these Examples and Experimental Examples are illustrative of the present invention, and the scope of the present invention is not limited to these Examples and Experimental Examples.

### [PREPARATION EXAMPLES]

Licochalcone A was purchased from Sigma-Aldrich (Product No. 68783, MO, USA) to perform the following experiments.

### [Examples]

### Example 1: Determination of Effects of Licochalcone A on Suppressing Adipocyte Differentiation and Suppressing Lipogenesis in Adipocytes

### (1) Incubation of Preadipocytes

3T3-L1 preadipocytes (ATCC No. CL-173) were purchased from the American Type Culture Collection (ATCC). 3T3-L1 preadipocytes were cultured in Dulbecco's Modified Eagle Medium (DMEM, Gibco) containing 10% fetal bovine serum (FBS), penicillin (100U/ml), and streptomycin (100U/ml) in a CO₂ incubator at 37 °C 5% CO₂.

### (2) Induction of Differentiation into Adipocytes

3T3-L1 cells were seeded in 6-well plate and treated with a hormone cocktail containing 0.5 mM dexamethasone, 10 µg/ml insulin, and 0.5 mM IBMX (3-isobutyl-1-methylxanthine) when the cells were post-confluent, and the medium was changed once every two days. The medium was replaced with 5 µg/ml insulin after 4 days and then replaced with normal medium after 2 days, thus inducing differentiation into adipocytes. During adipocyte differentiation, the cells were treated with 5 and 10 µm licochalcone A and treated with 10 µm pioglitazone as a positive control group.

### (3) Determination of Effects of Licochalcone A on Suppressing Adipocyte Differentiation: Observation of Morphology of Adipocytes

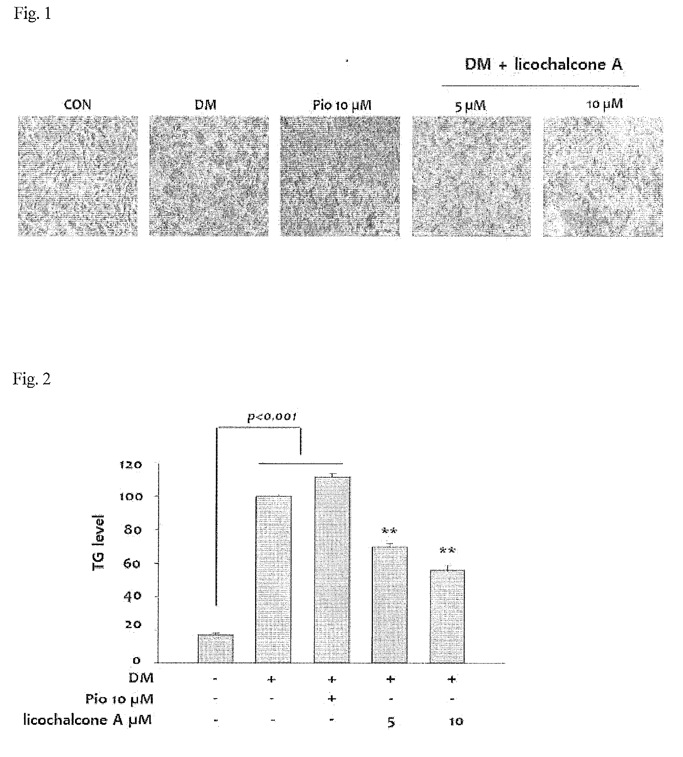

The differentiated adipocytes were washed with 1x PBS three times and fixed with 10% formaldehyde for 1 hour. Then, the resulting cells were stained with oil red O for 2 hours, washed with triple-distilled water, and observed under a microscope (FIG. 1). After removal of water from the cells, the cells were destained with 100% isopropanol, and then triglyceride levels were measured at 540 nm (FIG. 2).

As shown in FIGS. 1 and 2, it was confirmed that the licochalcone A suppresses the differentiation of adipocytes in a concentration-dependent manner.

### (4) Determination of Effects of Licochalcone A on Suppressing Expression of Transcription Factors for adipocyte differentiation: Protein Extraction, and Western Blotting

The differentiated 3T3-L1 adipocytes were homogenized with lysis buffer (50 mM tris-HCl, pH 7.5, 1mM EDTA, 0.25% sucrose, 0.4 mg/ml digonin, and 1.5 mM PMSF), and protein was centrifuged at 4 °C at 12,000 rpm for 20 minutes.

The protein concentration was measured using a Bio-Rad assay reagent (Bio-Rad, USA). 30 µg of the analyzed protein was resolved by SDS-PAGE, transferred to a gel membrane, blocked with 5% skim milk at room temperature for 1 hour, and then reacted overnight with primary antibodies diluted 1: 1000 (PPAR-γ, Santa cruz, 7273; C/EBPα, Santa cruz, 61, Actin, Santa cruz, 1616) at 4 °C overnight. Subsequently, the membrane was washed with Tris-buffered saline Tween-20 (TBST) three times and then reacted with secondary antibodies diluted 1:2000 (Santa Cruz, sc-2313; sc-2005; sc-2033) at room temperature for 1 hour.

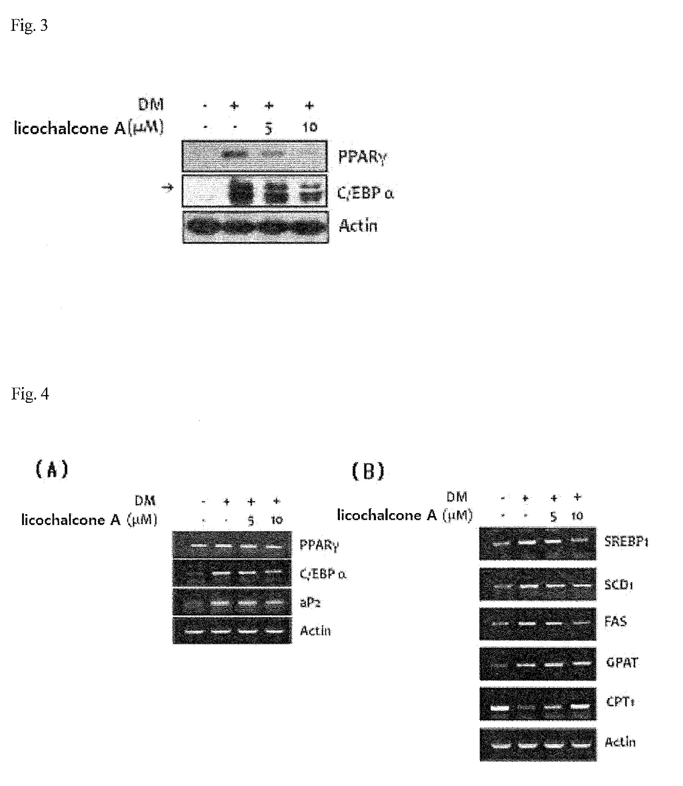

Then, the membrane was washed with TBST three times and exposed to X-ray film using an ECL solution (Amersham, Sweden). The results of Western blotting are shown in FIG. 3.

As shown in FIG. 3, it was confirmed that the licochalcone A inhibits the expression of transcription factors for adipocyte differentiation in a concentration-dependent manner, thus suppressing adipocyte differentiation.

### (5) Determination of Effects of Licochalcone A on Suppressing Expression of Transcription Factors for Adipocyte Differentiation and Lipolytic Genes: RNA Extraction, and RT-PCR

Total RNAs were extracted from the differentiated 3T3-L1 adipocytes using a guanidine thiocyanate-water saturated phenol/chloroform extraction procedure. The total RNAs in the water layer were precipitated with isopropyl alcohol and the optical density of the extracted RNA was measured at wavelengths of 260 nm and 280 nm and quantified. 1 µg of total RNA was reverse transcribed using Moloney murine leukemia virus reverse transcriptase and oligo (dT) 15 primers. The types and sequences of the primers are shown in Table 1.

**[Table 1] Primer sequence and annealing temperature**

| Gene | Forward primer (5' to 3') | Reverser primer (5' to 3') | Annealing temp (°C) |
|---|---|---|---|
| PPAR-γ | GCGCTACCGGTCTTCTATCA | TGCTGCCAAAAGACAAGCG | 57 |
| C/EBPα | GATCCTGGAACGAGAACAC | AGACTCGTGGAACACGGTGGT | 57 |
| aP2 | CGAGGGTTGGTTGTTGATCTGT | ATAGCACTGTTGGCCCTGGA | 57 |
| SREBP1 | GCGCTACCGGTCTTCTATCA | TGCTGCCAAAAGACAAGGG | 58 |
| SCD-1 | CCCTGAACATCGAGTGTCGA | CTTGCGCAGAGATTTGAGGTCCT | 57 |
| FAS | GGTAGTGGATACTCTGTCGTCCA | CATCAGCAACATCATTCGGT | 66 |
| GPAT | GGTAGTGGATACTCTGTCGTCCA | CAGCAACATCATTCGGT | 58 |
| CPT1 | GCTGGGCATGATTGCAAG | ACAGACTCCAGGTACCTGCTCAC | 60 |
| β-actin | GTCGTACCACTGGCATTGTG | GCCATCTCCTGCTCAAAGTC | 57 |

The primers were added to 25 µl reaction solution containing 20 mM tris-HCl (pH 8.4), 50 mM KCl, 1.5 mM MgCl₂, 0.5mM dNTP, 5 µl cDNA, and 2.5 units of Taq DNA polymerase such that each final concentration was set to 0.5 µM. The PCR was performed for 30 cycles with denaturation at 95 °C for 1 minute, annealing for 30 seconds (corresponding annealing temperatures are shown in Table 1), and extension at 72 °C for 1 minute. Then, the PCR products were electrophoresed at 100 V in a 1% agarose gel stained with 0.5 µg/ml ethidium bromide. β-actin was used as a control group of amplified genes. The results of RT-PCR are shown in FIG. 4.

As shown in FIG. 4, it can be seen that the licochalcone A suppresses adipocyte differentiation and lipogenesis and stimulates fatty acid oxidation in a concentration-dependent manner.

### Example 2: Determination of Effects of Licochalcone A on Suppressing Lipogenesis and Stimulating Lipolysis in Hepatocytes

### (1) Incubation of Hepatocytes

Human hepatoma HepG2 cells (ATCC No. HB-8065) were purchased from the American Type Culture Collection (ATCC). HepG2 cells were cultured in Dulbecco's Modified Eagle Medium (DMEM, Gibco) containing 10% fetal bovine serum (FBS), penicillin (100U/ml), and streptomycin (10OU/ml) in a CO₂ incubator at 37 °C 5% CO₂.

HepG2 cells were seeded in 6-well plate and treated the next day with licochalcone A at different concentrations (for 24 hours) and for different times such as 0, 6, 12, and 24 hours (treated with 20 µM licochalcone A), respectively, thus obtaining samples. Moreover, to determine the correlation between AMPK activation by licochalcone A and lipogenic/lipolytic mechanism, samples pretreated with 10 µM compound C (AMPK inhibitor) from Calbiochem (Darmstadt, Germany) were prepared.

### (2) Determination of Effects of Licochalcone A on AMPK Activation in HepG2 cells: Determination of AMPK and ACC Phosphorylation

The HepG2 cells were homogenized with lysis buffer (50 mM tris-HCl, pH 7.5, 1mM EDTA, 0.25% sucrose, 0.4 mg/ml digonin, and 1.5 mM PMSF), and protein was centrifuged at 4 °C at 12,000 rpm for 20 minutes.

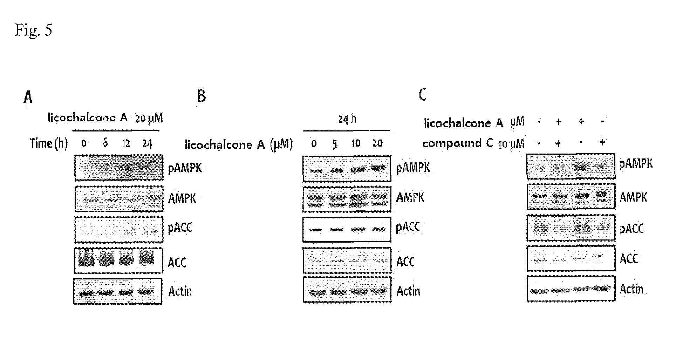

The protein concentration was measured using a Bio-Rad assay reagent (Bio-Rad, USA). 30 µg of the analyzed protein was resolved by SDS-PAGE, transferred to a gel membrane, blocked with 5% skim milk at room temperature for 1 hour, and then reacted overnight with primary antibodies diluted 1: 1000 (pAMPK: Cell signaling, 2531; AMPK: Cell signaling, 2532; pACC: Cell signaling, 3661; ACC: Cell signaling, 3662; Actin: Santa cruz, 1616) at 4 °C overnight. Subsequently, the membrane was washed with TBST three times and then reacted with secondary antibodies diluted 1:2000 (Santa Cruz, sc-2313; sc-2005; sc-2033) at room temperature for 1 hour. Then, the membrane was washed with TBST three times and exposed to X-ray film using an ECL solution (Amersham, Sweden). The results of Western blotting are shown in FIG. 5.

As can be seen from FIG. 5, the licochalcone A significantly phosphorylated AMPK and ACC in a dose-dependent and time-dependent manners. In particular, as can be seen from FIG. 5C, the AMPK and ACC phosphorylation was inhibited when HepG2 cells were pretreated with an AMPK inhibitor, compound C, from which it can be seen that the licochalcone A may act through AMPK pathway.

### (3) Determination of Effects of Licochalcone A on Suppressing Expression of Transcription Factor for Lipogenesis (SREBP1) and Stimulating Expression of Lipolytic Genes: RNA Extraction, and RT-PCR

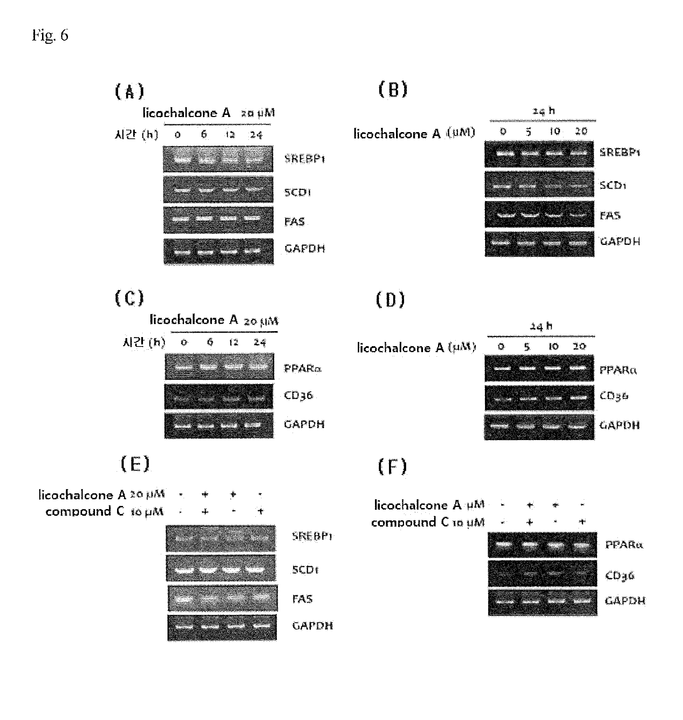

The RT-PCR was performed by the same method as Example 1(5), except that the human hepatoma HepG2 cells obtained above and the primer sequences and annealing conditions of Table 2 were used. The results of RT-PCR are shown in FIG. 6.

**[Table 2] Primer sequence and annealing temperature**

| Gene | Forward primer (5' to 3') | Reverser primer (5' to 3') | Annealing temp (°C) |
|---|---|---|---|
| SREBP1 | GTGGCGGCTGCATTGAGAGTGAG | AGGTACCCGAGGGCATCCGAGAAT | 62 |
| SCD-1 | CCCTGAACATCGAGTGTCGA | CTTGCCCAGAGATTTGAGGTCCT | 57 |
| FAS | GGTAGTGGATACTCTGTCGTCCA | CATCAGCAACATCATTCGGT | 66 |
| PPARα | TCCGACTCCGTCTTCTTGAT | GCCTAAGGAAACCGTTCTGTG | 54 |
| CD36 | GGGCTATAGGGATCCATTTTT | CCTTTCAGATTAACGTCGGATTC | 50 |
| GAPDH | TCCACCACCCTGTTGCTGTA | AGGTACCCGAGGGCATCCGAGAAT | 54 |

As shown in FIG. 6, it can be seen that the expression of transcription factors for lipogenesis (SREBP1, SCD-1, and FAS) was reduced by the licochalcone A for different times and at different concentrations (FIGS. 6A and 6B) and the levels of lipolytic genes (PPARα and CD36) were increased (FIGS. 6C and 6D).

Moreover, it was confirmed that when the HePG2 cells were pretreated with an AMPK inhibitor, compound C, to investigate whether the effects of licochalcone A on lipogenic and lipolytic genes are associated with AMPK, the levels of lipogenic genes, SREBP-1, SCD1, and FAS, were increased (FIG. 6E) and the levels of lipolytic genes were reduced (FIG. 6F), from which it can be seen that the licochalcone A may act through AMPK pathway.

The invention has been described in detail with reference to preferred embodiments thereof. However, it will be appreciated by those skilled in the art that changes may be made in these embodiments without departing from the principles and spirit of the invention, the scope of which is defined in the appended claims and their equivalents.

## Claims

1. A pharmaceutical composition containing licochalcone A as an active ingredient for preventing or treating AMP-activated protein kinase (AMPK)-related diseases.

2. The pharmaceutical composition of claim 1, wherein the AMPK-related diseases are selected from the group consisting of lipid metabolism disorder, hyperlipidemia, metabolic syndrome, obesity, fatty liver disease, and degenerative diseases.

3. The pharmaceutical composition of claim 1 or 2, wherein the licochalcone A is contained in an amount of 1 to 50 wt% with respect to the total weight of the composition.

4. A food composition containing licochalcone A as an active ingredient for preventing or improving AMPK-related diseases.

5. The food composition of claim 4, wherein the AMPK-related diseases are selected from the group consisting of lipid metabolism disorder, hyperlipidemia, metabolic syndrome, obesity, fatty liver disease, and degenerative diseases.

6. The food composition of claim 4 or 5, wherein the licochalcone A is contained in an amount of 1 to 50 wt% with respect to the total weight of the composition.

7. A method for preventing or treating AMPK-related diseases, the method comprising administering a therapeutically effective amount of licochalcone A to a subject in need of preventing or treating the above-mentioned AMPK-related diseases.
